# EUROPEAN PATENT APPLICATION

(11) **EP 1 179 590 A1**
(43) Date of publication of application: **13.02.2002**
(21) Application number: 00922907.1
(22) Date of filing: 28.04.2000
(51) Int. Cl.: C12N 15/12, C07K 14/435, C12N 1/15, C12N 1/19, C12N 1/21, C12N 5/10, C12P 21/02, C07K 16/18, G01N 33/53, G01N 33/50, A01K 67/027

(54) **MEG-3 PROTEIN**

(30) Priority: 30.04.1999 JP 12356199
(71) Applicant: Kurokawa, Kiyoshi, Sinjuku-ku, Tokyo 162-0061 (JP); Miyata, Toshio, Isehara-shi, Kanagawa 259-1132 (JP)
(72) Inventor: MIYATA, Toshio, Isehara-shi, Kanagawa 259-1117 (JP)
(74) Representative: Grünecker, August, Dipl.-Ing.
(86) International application number: JP0002831
(87) International publication number: WO0066729

(57) **Abstract**

The present invention provides a DNA expressed at high frequency in mesangial cells and a protein (Meg-3) encoded by this DNA. These substances are useful in, for example, identifying mesangial cells and detecting abnormalities in mesangial cells. Moreover, the above protein would be helpful for clarification of the functions of mesangial cells and, in its turn, for clarification of the causes of diseases relating to mesangial cells. This protein is expectedly applicable to the treatment, diagnosis, and such of diseases relating to mesangial cells.

SEQUENCE LISTING

<110> MIYATA, Toshio
      KUROKAWA, Kiyoshi

<120> Meg-3 protein

<130> KRK-103PCT

<140>
<141>

<150> JP 1999-123561
<151> 1999-04-30

<160> 8

<170> PatentIn Ver. 2.1

<210> 1
<211> 3768
<212> DNA
<213> Homo sapiens

<220>
<221> CDS

<221> CDS
<222> (53)..(2251)

<400> 1
caggaactgg gccagctccg gtcccttcct tttggggctc tcactctgga gg atg ggg 58
Met Gly
1

tgg atg gga gaa aaa acc ggg aag atc ctg acg gag ttc ctc cag ttc 106
Trp Met Gly Glu Lys Thr Gly Lys Ile Leu Thr Glu Phe Leu Gln Phe
        5                   10                  15

tat gaa gac cag tat ggc gtg gct ctc ttc aac agc atg cgc cat gag 154
Tyr Glu Asp Gln Tyr Gly Val Ala Leu Phe Asn Ser Met Arg His Glu
    20                  25                  30

att gag ggc acg ggg ctg ccg cag gcc cag ctg ctc tgg cgc aag gtg 202
Ile Glu Gly Thr Gly Leu Pro Gln Ala Gln Leu Leu Trp Arg Lys Val
35                  40                  45                  50

cca ctg gac gag cgc atc gtc ttc tcg ggg aac ctc ttc cag cac cag 250
Pro Leu Asp Glu Arg Ile Val Phe Ser Gly Asn Leu Phe Gln His Gln
                55                  60                  65

gag gac agc aag aag tgg aga aac cgc ttc agc ctc gtg ccc cac aac 298
Glu Asp Ser Lys Lys Trp Arg Asn Arg Phe Ser Leu Val Pro His Asn

            70                  75                  80

tac ggg ctg gtg ctc tac gaa aac aaa gcg gcc tat gag cgg cag gtc 346
Tyr Gly Leu Val Leu Tyr Glu Asn Lys Ala Ala Tyr Glu Arg Gln Val
        85                  90                  95

cca cca cga gcc gtc atc aac agt gca ggc tac aaa atc ctc acg tcc 394
Pro Pro Arg Ala Val Ile Asn Ser Ala Gly Tyr Lys Ile Leu Thr Ser
    100                 105                 110

gtg gac caa tac ctg gag ctc att ggc aac tcc tta cca ggg acc acg 442
Val Asp Gln Tyr Leu Glu Leu Ile Gly Asn Ser Leu Pro Gly Thr Thr
115                 120                 125                 130

gca aag tcg ggc agt gcc ccc atc ctc aag tgc ccc aca cag ttc ccg 490
Ala Lys Ser Gly Ser Ala Pro Ile Leu Lys Cys Pro Thr Gln Phe Pro
                135                 140                 145

ctc atc ctc tgg cat cct tat gcg cgt cac tac tac ttc tgc atg atg 538
Leu Ile Leu Trp His Pro Tyr Ala Arg His Tyr Tyr Phe Cys Met Met
            150                 155                 160

aca gaa gcc gag cag gac aag tgg cag gct gtg ctg cag gac tgc atc 586
Thr Glu Ala Glu Gln Asp Lys Trp Gln Ala Val Leu Gln Asp Cys Ile
        165                 170                 175

cgg cac tgc aac aat gga atc cct gag gac tcc aag gta gag ggc cct 634
Arg His Cys Asn Asn Gly Ile Pro Glu Asp Ser Lys Val Glu Gly Pro
    180                 185                 190

gcg ttc aca gat gcc atc cgc atg tac cga cag tcc aag gag ctg tac 682
Ala Phe Thr Asp Ala Ile Arg Met Tyr Arg Gln Ser Lys Glu Leu Tyr
195                 200                 205                 210

ggc acc tgg gag atg ctg tgt ggg aac gag gtg cag atc ctg agc aac 730
Gly Thr Trp Glu Met Leu Cys Gly Asn Glu Val Gln Ile Leu Ser Asn
                215                 220                 225

ctg gtg atg gag gag ctg ggc cct gag ctg aag gca gag ctc ggc ccg 778
Leu Val Met Glu Glu Leu Gly Pro Glu Leu Lys Ala Glu Leu Gly Pro
            230                 235                 240

cgg ctg aag ggg aaa ccg cag gag cgg cag cgg cag tgg atc cag atc 826
Arg Leu Lys Gly Lys Pro Gln Glu Arg Gln Arg Gln Trp Ile Gln Ile
        245                 250                 255

tcg gac gcc gtg tac cac atg gtg tac gag cag gcc aag gcg cgc ttc 874
Ser Asp Ala Val Tyr His Met Val Tyr Glu Gln Ala Lys Ala Arg Phe
    260                 265                 270

gag gag gtg ctg tcc aag gtg cag cag gtg cag ccg gcc atg cag gcc 922
Glu Glu Val Leu Ser Lys Val Gln Gln Val Gln Pro Ala Met Gln Ala

275                 280                 285                 290

gtc atc cga act gac atg gac caa att atc acc tcc aag gag ctc ctt 970
Val Ile Arg Thr Asp Met Asp Gln Ile Ile Thr Ser Lys Glu Leu Leu
                295                 300                 305

gcc agc aag atc cga gcc ttc atc ctc ccc aag gca gag gtg tgc gtg 1018
Ala Ser Lys Ile Arg Ala Phe Ile Leu Pro Lys Ala Glu Val Cys Val
            310                 315                 320

cgg aac cat gtc cag ccc tac atc cca tcc atc ctg gag gcc ctg atg 1066
Arg Asn His Val Gln Pro Tyr Ile Pro Ser Ile Leu Glu Ala Leu Met
        325                 330                 335

gtc ccc acc agc cag ggc ttc act gag gtg cga gat gtc ttc ttc aag 1114
Val Pro Thr Ser Gln Gly Phe Thr Glu Val Arg Asp Val Phe Phe Lys
    340                 345                 350

gag gtc acg gac atg aac ctg aac gtc atc aac gag ggc ggc att gac 1162
Glu Val Thr Asp Met Asn Leu Asn Val Ile Asn Glu Gly Gly Ile Asp
355                 360                 365                 370

aag ctg ggc gag tac atg gag aag ctg tcc cgg ctg gcg tac cac ccc 1210
Lys Leu Gly Glu Tyr Met Glu Lys Leu Ser Arg Leu Ala Tyr His Pro
                375                 380                 385

ctg aag atg cag agc tgc tat gag aag atg gag tcg ctg cga ctg gac 1258
Leu Lys Met Gln Ser Cys Tyr Glu Lys Met Glu Ser Leu Arg Leu Asp
            390                 395                 400

ggg ctg cag cag cga ttt gat gtg tcc agc acg tcc gtg ttc aag cag 1306
Gly Leu Gln Gln Arg Phe Asp Val Ser Ser Thr Ser Val Phe Lys Gln
        405                 410                 415

cga gcc cag atc cac atg cgg gag caa atg gac aat gcc gtg tat acg 1354
Arg Ala Gln Ile His Met Arg Glu Gln Met Asp Asn Ala Val Tyr Thr
    420                 425                 430

ttc gag acc ctc ctg cac cag gag ctg ggg aag ggg ccc acc aag gag 1402
Phe Glu Thr Leu Leu His Gln Glu Leu Gly Lys Gly Pro Thr Lys Glu
435                 440                 445                 450

gag ctg tgc aag tcc atc cag cgg gtc ctg gag cgg gtg ctg aaa aaa 1450
Glu Leu Cys Lys Ser Ile Gln Arg Val Leu Glu Arg Val Leu Lys Lys
                455                 460                 465

tac gac tac gac agc agc tct gtg cgg aag agg ttc ttc cgg gag gcg 1498
Tyr Asp Tyr Asp Ser Ser Ser Val Arg Lys Arg Phe Phe Arg Glu Ala
            470                 475                 480

ctg ctg cag atc agc atc ccg ttc ctg ctc aag aag ctg gcc cct acc 1546
Leu Leu Gln Ile Ser Ile Pro Phe Leu Leu Lys Lys Leu Ala Pro Thr

        485                 490                 495

tgc aag tcg gag ctg ccc cgg ttc cag gag ctg atc ttc gag gac ttt 1594
Cys Lys Ser Glu Leu Pro Arg Phe Gln Glu Leu Ile Phe Glu Asp Phe
    500                 505                 510

gcc agg ttc atc ctg gtg gaa aac acg tac gag gag gtg gtg ctg cag 1642
Ala Arg Phe Ile Leu Val Glu Asn Thr Tyr Glu Glu Val Val Leu Gln
515                 520                 525                 530

acc gtc atg aag gac atc ctg cag gct gtg aag gag gcc gcg gtg cag 1690
Thr Val Met Lys Asp Ile Leu Gln Ala Val Lys Glu Ala Ala Val Gln
                535                 540                 545

agg aag cac aac ctc tac cgg gac agc atg gtc atg cac aac agc gac 1738
Arg Lys His Asn Leu Tyr Arg Asp Ser Met Val Met His Asn Ser Asp
            550                 555                 560

ccc aac ctg cac ctg ctg gcc gag ggc gcc ccc atc gac tgg ggc gag 1786
Pro Asn Leu His Leu Leu Ala Glu Gly Ala Pro Ile Asp Trp Gly Glu
        565                 570                 575

gag tac agc aac agc ggc ggg ggc ggc agc ccc agc ccc agc acc ccg 1834
Glu Tyr Ser Asn Ser Gly Gly Gly Gly Ser Pro Ser Pro Ser Thr Pro
    580                 585                 590

gag tca gcc acc ctc tcg gaa aag cga cgg cgc gcc aag cag gtg gtc 1882
Glu Ser Ala Thr Leu Ser Glu Lys Arg Arg Arg Ala Lys Gln Val Val
595                 600                 605                 610

tct gtg gtc cag gat gag gag gtg ggg ctg ccc ttt gag gct agc cct 1930
Ser Val Val Gln Asp Glu Glu Val Gly Leu Pro Phe Glu Ala Ser Pro
                615                 620                 625

gag tca cca cca cct gcg tcc ccg gac ggt gtc act gag atc cga ggc 1978
Glu Ser Pro Pro Pro Ala Ser Pro Asp Gly Val Thr Glu Ile Arg Gly
            630                 635                 640

ctg ctg gcc caa ggt ctg cgg cct gag agc ccc cca cca gcc ggc ccc 2026
Leu Leu Ala Gln Gly Leu Arg Pro Glu Ser Pro Pro Pro Ala Gly Pro
        645                 650                 655

ctg ctc aac ggg gcc ccc gct ggg gag agt ccc cag cct aag gcc gcc 2074
Leu Leu Asn Gly Ala Pro Ala Gly Glu Ser Pro Gln Pro Lys Ala Ala
    660                 665                 670

ccc gag gcc tcc tcg ccg cct gcc tca ccc ctc cag cat ctc ctg cct 2122
Pro Glu Ala Ser Ser Pro Pro Ala Ser Pro Leu Gln His Leu Leu Pro
675                 680                 685                 690

gga aag gct gtg gac ctt ggg ccc ccc aag ccc agc gac cag gag act 2170
Gly Lys Ala Val Asp Leu Gly Pro Pro Lys Pro Ser Asp Gln Glu Thr

                695                 700                 705

gga gag cag gtg tcc agc ccc agc agc cac ccc gcc ctc cac acc acc 2218
Gly Glu Gln Val Ser Ser Pro Ser Ser His Pro Ala Leu His Thr Thr
            710                 715                 720

acc gag gac agt gca ggg gtg cag act gag ttc taggccagtg ggtccctgac 2271
Thr Glu Asp Ser Ala Gly Val Gln Thr Glu Phe
        725                 730

tgctgcacat ggcacaggcc gttcccttcc ggacccaggc aggctcagct ctggggaggg 2331

caccctggtc tgtgccttgt gggtggaggc ggggcagggc tgtgtggcac cgccagggag 2391

cgggcccacc tgagtcactt tattgggttc agtcaacact ttcttgctcc ctgttttctc 2451

ttctgtggga tgatctcaga tgcaggggct ggttttgggg ttttcctgct tgtgccaagg 2511

gctggacact gctgggggc tggaaagccc ctcccttcct gtccttctgt ggcctccatc 2571

ccctcatggg tgctgccatc cttcctggag agagggaggt gaaagctggt gtgagcccag 2631

tgggttcccg cccactcacc caggagctgg ctgggccagg accgggagag ggagcactgc 2691

tgccctcctg gccctgctcc ttccgcagtt aggggtggac cgagcctcgc tttccccact 2751

gttctggagg gaaggggaag gagggggtct tcaggctgga gccaggctgg gggtgctggg 2811

tggagagatg agatttaggg ggtgcctcat ggggtgggca ggcctggggt gaaatgagaa 2871

aggcccagaa cgtgcaggtc tgcggagggg aagtgtcctg agtgaaggag gggaccccat 2931

cctggggatg ctgggagtga gtgagtgaga tggctgagtg agggttatgg ggagcctgag 2991

gttttatggg cctgtgtatc cccttctccc ggccccagcc tgcctccctc ctgcccgcct 3051

ggcccacagg tctccctctg gtccctgtcc ctctggtggt tggggatgga gcggcagcaa 3111

ggggtgtaat ggggctgggt tctgtcttct acaggccacc ccgaggtcct cagtggttgc 3171

ctggggagcc ggacggggct cctgaggggt acaggttggg tgggccctcc ctgagggtct 3231

ggggtcaggc tttggcctct gctgcctctc agtcaccaag tcacctccct ctgaaaatcc 3291

agtcccttct ttggatgtcc ttgtgagtca ctctgggcct ggctgtcgtc cctcctcagc 3351

ttcttgttcc tgggacaagg gtcaagccag gatgggccca ggcntgggat cccccacccc 3411

aggaccccac aggccccctc ccctgntgnt ttgcgggggg cagggcagaa atggactcct 3471

tttgggtccc cgaggtgggg tcccctccca gccctgcatc ctccgtgccc tagacctgct 3531

ccccagagga ggggccttga cccacaggaa gtgtggtggc gcctggcaat cagggacccc 3591

cagctgccgc agccctggtt tttggcgcat cttttccctc ttgtcccgaa gatttgcgcc 3651

tttagtgcct tttgaggggt tcccatcatc cctccctgat attgtattga aaatattatg 3711

cacactgttc atgcttttac taatcaataa acgctttatt taaaaaaaaa aaaaaaa 3768

<210> 2

<211> 733

<212> PRT

<213> Homo sapiens

<400> 2

Met Gly Trp Met Gly Glu Lys Thr Gly Lys Ile Leu Thr Glu Phe Leu
1               5                   10                  15

Gln Phe Tyr Glu Asp Gln Tyr Gly Val Ala Leu Phe Asn Ser Met Arg
            20                  25                  30

His Glu Ile Glu Gly Thr Gly Leu Pro Gln Ala Gln Leu Leu Trp Arg
        35                  40                  45

Lys Val Pro Leu Asp Glu Arg Ile Val Phe Ser Gly Asn Leu Phe Gln
    50                  55                  60

His Gln Glu Asp Ser Lys Lys Trp Arg Asn Arg Phe Ser Leu Val Pro
65                  70                  75                  80
His Asn Tyr Gly Leu Val Leu Tyr Glu Asn Lys Ala Ala Tyr Glu Arg
                85                  90                  95
Gln Val Pro Pro Arg Ala Val Ile Asn Ser Ala Gly Tyr Lys Ile Leu
            100                 105                 110
Thr Ser Val Asp Gln Tyr Leu Glu Leu Ile Gly Asn Ser Leu Pro Gly
        115                 120                 125
Thr Thr Ala Lys Ser Gly Ser Ala Pro Ile Leu Lys Cys Pro Thr Gln
    130                 135                 140
Phe Pro Leu Ile Leu Trp His Pro Tyr Ala Arg His Tyr Tyr Phe Cys
145                 150                 155                 160
Met Met Thr Glu Ala Glu Gln Asp Lys Trp Gln Ala Val Leu Gln Asp
                165                 170                 175
Cys Ile Arg His Cys Asn Asn Gly Ile Pro Glu Asp Ser Lys Val Glu
            180                 185                 190
Gly Pro Ala Phe Thr Asp Ala Ile Arg Met Tyr Arg Gln Ser Lys Glu

        195                 200                 205

Leu Tyr Gly Thr Trp Glu Met Leu Cys Gly Asn Glu Val Gln Ile Leu

    210                 215                 220

Ser Asn Leu Val Met Glu Glu Leu Gly Pro Glu Leu Lys Ala Glu Leu

225                 230                 235                 240

Gly Pro Arg Leu Lys Gly Lys Pro Gln Glu Arg Gln Arg Gln Trp Ile

                245                 250                 255

Gln Ile Ser Asp Ala Val Tyr His Met Val Tyr Glu Gln Ala Lys Ala

            260                 265                 270

Arg Phe Glu Glu Val Leu Ser Lys Val Gln Gln Val Gln Pro Ala Met

        275                 280                 285

Gln Ala Val Ile Arg Thr Asp Met Asp Gln Ile Ile Thr Ser Lys Glu

    290                 295                 300

Leu Leu Ala Ser Lys Ile Arg Ala Phe Ile Leu Pro Lys Ala Glu Val

305                 310                 315                 320

Cys Val Arg Asn His Val Gln Pro Tyr Ile Pro Ser Ile Leu Glu Ala

                325                 330                 335

Leu Met Val Pro Thr Ser Gln Gly Phe Thr Glu Val Arg Asp Val Phe
            340                 345                 350
Phe Lys Glu Val Thr Asp Met Asn Leu Asn Val Ile Asn Glu Gly Gly
        355                 360                 365
Ile Asp Lys Leu Gly Glu Tyr Met Glu Lys Leu Ser Arg Leu Ala Tyr
    370                 375                 380
His Pro Leu Lys Met Gln Ser Cys Tyr Glu Lys Met Glu Ser Leu Arg
385                 390                 395                 400
Leu Asp Gly Leu Gln Gln Arg Phe Asp Val Ser Ser Thr Ser Val Phe
                405                 410                 415
Lys Gln Arg Ala Gln Ile His Met Arg Glu Gln Met Asp Asn Ala Val
            420                 425                 430
Tyr Thr Phe Glu Thr Leu Leu His Gln Glu Leu Gly Lys Gly Pro Thr
        435                 440                 445
Lys Glu Glu Leu Cys Lys Ser Ile Gln Arg Val Leu Glu Arg Val Leu
    450                 455                 460
Lys Lys Tyr Asp Tyr Asp Ser Ser Ser Val Arg Lys Arg Phe Phe Arg
465                 470                 475                 480

Glu Ala Leu Leu Gln Ile Ser Ile Pro Phe Leu Leu Lys Lys Leu Ala
                485                 490                 495
Pro Thr Cys Lys Ser Glu Leu Pro Arg Phe Gln Glu Leu Ile Phe Glu
            500                 505                 510
Asp Phe Ala Arg Phe Ile Leu Val Glu Asn Thr Tyr Glu Glu Val Val
        515                 520                 525
Leu Gln Thr Val Met Lys Asp Ile Leu Gln Ala Val Lys Glu Ala Ala
    530                 535                 540
Val Gln Arg Lys His Asn Leu Tyr Arg Asp Ser Met Val Met His Asn
545                 550                 555                 560
Ser Asp Pro Asn Leu His Leu Leu Ala Glu Gly Ala Pro Ile Asp Trp
                565                 570                 575
Gly Glu Glu Tyr Ser Asn Ser Gly Gly Gly Gly Ser Pro Ser Pro Ser
            580                 585                 590
Thr Pro Glu Ser Ala Thr Leu Ser Glu Lys Arg Arg Arg Ala Lys Gln
        595                 600                 605
Val Val Ser Val Val Gln Asp Glu Glu Val Gly Leu Pro Phe Glu Ala

    610                 615                 620

Ser Pro Glu Ser Pro Pro Pro Ala Ser Pro Asp Gly Val Thr Glu Ile

625                 630                 635                 640

Arg Gly Leu Leu Ala Gln Gly Leu Arg Pro Glu Ser Pro Pro Pro Ala

                645                 650                 655

Gly Pro Leu Leu Asn Gly Ala Pro Ala Gly Glu Ser Pro Gln Pro Lys

            660                 665                 670

Ala Ala Pro Glu Ala Ser Ser Pro Pro Ala Ser Pro Leu Gln His Leu

        675                 680                 685

Leu Pro Gly Lys Ala Val Asp Leu Gly Pro Pro Lys Pro Ser Asp Gln

    690                 695                 700

Glu Thr Gly Glu Gln Val Ser Ser Pro Ser Ser His Pro Ala Leu His

705                 710                 715                 720

Thr Thr Thr Glu Asp Ser Ala Gly Val Gln Thr Glu Phe

                725                 730

<211> 18

<212> DNA

<213> Artificial Sequence

<220>

<223> Description of Artificial Sequence:Artificially
      Synthesized Primer Sequence

<400> 3
tgtaaaacga cggccagt                                                    18

<210> 4

<211> 21

<212> DNA

<213> Artificial Sequence

<220>

<223> Description of Artificial Sequence:Artificially
      Synthesized Primer Sequence

<400> 4
accatgatta cgccaagctt g                                                21

<210> 5

<211> 30

<212> DNA

<213> Artificial Sequence

<220>

<223> Description of Artificial Sequence:Artificially

Synthesized Probe Sequence

<400> 5

tacctggagc tcattggcaa ctccttacca 30

<210> 6

<211> 22

<212> DNA

<213> Artificial Sequence

<220>

<223> Description of Artificial Sequence:Artificially

Synthesized Primer Sequence

<400> 6

cggaattcat ggggtggatg gg 22

<210> 7

<211> 31

<212> DNA

<213> Artificial Sequence

<220>

<223> Description of Artificial Sequence:Artificially

Synthesized Primer Sequence

<400> 7

gcgaattcta gaactcagtc tgcacccctg c 31

<210> 8

<211> 29

<212> DNA

<213> Artificial Sequence

<220>

<223> Description of Artificial Sequence:Artificially

Synthesized Primer Sequence

<400> 8

gcgaattcga actcagtctg cacccctgc 29

## Description

### Technical Field

The present invention belongs to the field of genetic engineering and specifically relates to isolation of a gene of renal cells.

### Background Art

Sixty trillion various cells *in vivo* essentially comprise identical genomic DNA. For the normal physiological functions, the expression of these genes is strictly controlled by signals received by cell lines and cells. Therefore, elucidation of genes expressed in each cell type is very important.

A mesangial cell plays a pivotal role in maintaining the structure and function of a glomerulus and also has a central meaning of pathophysiology for each type of nephritis. For example, proliferation of mesangial cells and accumulation of extracellular mesangial matrix are thought to be important pathological finding of glomerulosclerosis in a patient suffering from various glomerular diseases such as chronic nephritis and diabetic nephropathy. Therefore, identification of genes expressed specifically in mesangial cells and elucidation of its function are helpful for understanding biological characteristics of mesangial cells and the causes of diseases relating to mesangial cells, and in turn, treating or diagnosing diseases relating to mesangial cells.

Thyl antigen is known as a marker for mesangial cells in rats. However, this gene is not specific to mesangial cells and is not expressed in human mesangial cells (Miyata T. et al., Immunology, 1989, 67: 531-533; and Miyata T. et al., Immunology, 1990, 69: 391-395). Mesangial cells are known to express α smooth muscle actin when activated, but this gene is also not specific to mesangial cells. Any genes characteristically in mesangial cells have not been reported.

The present inventor has previously reported MEGSIN as a protein that is expressed specifically in the mesangial cells (J. Clin. Invest, 1998 Aug 15, 120 : 4, 828-36). The present invention relates to a novel protein having a structure that is distinctly different from the MEGSIN.

### Disclosure of the Invention

An objective of the present invention is to isolate a gene highly expressed in mesangial cells.

The current inventor isolated mRNA from in *vitro* cultures of human mesangial cells to construct a cDNA library of 3' side. Then, numerous clones in cDNA library were randomly selected, and determined their sequences. Next, determined sequences were compared with the known nucleotide sequences of cDNA clones of 3' side obtained from various organs and cells to determine the clones expressed in mesangial cells. Then, the λZIPLox cDNA library prepared from mesangial cells using the insert of this clone as a probe was screened to determine the nucleotide sequence (3768 bp) of the positive clone, and thus the present invention was completed. Based on the determined nucleotide sequence, the amino acid sequence of the longest open reading frame with the Kozak translation initiation codon was determined. The protein of this invention having this deduced amino acid sequence was named Meg-3 by the present inventor. The nucleotide sequence of human Meg-3 cDNA and the deduced amino acid sequence for human Meg-3 are shown in SEQ ID NO: 1 and SEQ ID NO: 2, respectively. No other sequence than that of the EST, which has 90% or more identity with the nucleotide sequence at 300 to 500 bases from the 3'end of the nucleotide sequence described in SEQ ID NO: 1, were found as nucleotide sequences sharing homology with the nucleotide sequence described in SEQ ID NO: 1.

An amino acid sequence homology search performed on this amino acid sequence using the SwissProt database confirmed that Meg-3 is a novel protein. Moreover, motif search on the deduced amino acid sequence of Meg-3 was conducted which revealed an amino acid sequence that closely resembles many proteins called proline rich protein, at regions following after the 500th amino acid counted from the N-terminus. The region comprising the 81 amino acid residues from 621st amino acid to 701st amino acid is especially abound in proline (27.2%), and possess at two points the amino acid sequence (xPESPPPAxP), which resembles the amino acid sequence (xPxxPPPFxP) of the proline rich peptide (PR peptide) that binds to the SH3 (Src homology 3) domain. These finding suggest the possibility that the C-terminus structure of the Meg-3 protein may bind as a PR domain to the SH3 domain of intracellular signal transduction substance, like those belonging to the Src family and such, and that it may be related to the signal transduction pathway. The high possibility (52.2%) of the protein having the amino acid sequence of SEQ ID NO: 2 to localize in the cytoplasm also indicates the possibility that the Meg-3 may be related to signal transduction. No other amino acid sequence having specifically high identity to other regions (amino acids 1 to 550 from the N-terminus) than that above could be found.

One of the characteristics in the human primary cell culture is that the Meg-3 gene is especially highly expressed in mesangial cells. In other primary cell cultures, expression in renal cortical epithelial cell and fibroblast are observed, and slight expression in human endothelial cell of the umbilical vein and smooth muscle cells are observed. Furthermore, when the topography of Meg-3 was detected by Northern blotting, high expression of Meg-3 was observed in the placenta followed by the expression in the pancreas. Additionally, weak expression was observed in kidney, lung and heart, and expression of Meg-3 was hardly observed in liver and skeletal muscles. No expression of Meg-3 could be detected in the brain. This invention was completed based on these findings.

This invention specifically includes the following protein, DNA and their uses:
(1) A protein comprising the amino acid sequence of SEQ ID NO: 2, or a protein comprising the amino acid sequence of SEQ ID NO: 2 in which one or more amino acids are replaced, deleted, added, and/or inserted, and being functionally equivalent to the protein comprising the amino acid sequence of SEQ ID NO: 2.
(2) The protein of (1), wherein the protein comprises the amino acid sequence of SEQ ID NO: 2.
(3) A DNA encoding the protein of (1).
(4) The DNA of (3), wherein the DNA comprises the nucleotide sequence of SEQ ID NO: 1.
(5) A DNA encoding the protein of (1) or functionally equivalent with these protein, the DNA hybridizing under stringent conditions with DNA comprising the nucleotide sequence of SEQ ID NO: 1.
(6) A DNA hybridizing specifically with the DNA of (4) and having a chain length of at least 15 nucleotides.
(7) An antisense DNA against the DNA of (4) or a portion thereof.
(8) A vector comprising the DNA of any one of (3), (4) and (5).
(9) A transformant expressibly carrying the DNA of any one of (3), (4) and (5).
(10) A method for producing the protein of (1), the method comprising culturing the transformant of (9) and collecting an expression product of the DNA of any one of (3), (4) and (5).
(11) A reagent for detecting mesangial cells comprising the DNA of (6).
(12) An antibody binding to the protein of (1).
(13) The antibody of (12), wherein the antibody recognizes a portion of a protein comprising an amino acid sequence selected from the amino acid sequence of SEQ ID NO: 2.
(14) The antibody of (13), wherein the antibody is a monoclonal antibody.
(15) An immunoassay method for measuring the protein of (2) or a fragment thereof based on immunological binding of the antibody of any one of (13) or (14) to the protein of (2) or a fragment thereof.
(16) A reagent for detecting the mesangial cell, the reagent comprising the antibody of any one of (12) to (14).
(17) A method for detecting mesangial proliferative nephropathy, the method comprising measuring the protein of (2) or a fragment thereof contained in a biological sample and comparing the measured value with that obtained from a normal sample.
(18) A transgenic nonhuman vertebrate in which the expression level of a gene encoding Meg-3 is modified.
(19) The transgenic nonhuman vertebrate of (18), wherein the nonhuman vertebrate is a mouse.
(20) The transgenic nonhuman vertebrate of (19), wherein the nonhuman vertebrate is a knockout mouse in which the expression of a gene encoding Meg-3 is inhibited.

To fulfill the issues mentioned above, the present inventor used a 3'-directed cDNA library. This method avoids the variation of cloning efficiency affected by size of DNA. The sequence at the 3' region characterizes each of the genes, and the sequence data of approximately 200 to 300 bp are large enough to demonstrate the characteristics of the gene (Yasuda Y., Miyata T. et al., Kidney Int, 1998 Jan, 53:1, 154-8).

The DNA encoding human Meg-3 of the present invention can be obtained by preparing mRNA from mesangial cells and converting them to the double stranded cDNA by the known methods. mRNA can be prepared by, for example, the guanidine isothiocyanate-cesium chloride method (Chirwin, et al., Biochemistry 18, 5294, 1979), and the treatment with a surfactant and phenol in the presence of deoxyribonuclease (Berger & Birkenmeier, Biochemistry 18, 5143, 1979), etc. Preparation of poly (A)⁺ RNA from total RNA can be performed by, for example, the affinity chromatography using such a carrier bound to oligo(dT) as Sepharose, cellulose, latex particles, etc. The cDNA (1st strand) can be obtained by treating the above-mentioned obtained mRNA as the template and the oligo (dT), which is complementary to the poly (A) chain at the 3'-terminus, the random primer or the synthetic oligonucleotide, which corresponds to part of the amino acid sequence of Meg-3, as the primer with a reverse transcriptase. The mRNA in the hybrid composed of mRNA and cDNA complementary thereto is partially cleaved with the *E.coli* RNase H. Using the cleaved mRNA as the primer, cDNA (2nd strand) is synthesized by *E.coli* DNA polymerase I. Finally, the double strand cDNA can be obtained by treating with *E.coli* DNA ligase.

The DNA can be cloned by RT-PCR method using poly (A)⁺ RNA from mesangial cells as a template, and primers synthesized based on the human Meg-3 gene nucleotide sequence. Alternatively, without using PCR, the target cDNA can be obtained by directly screening a cDNA library with a probe synthesized based on human Meg-3 gene nucleotide sequence. The gene of the present invention can be selected by confirming the nucleotide sequence of the gene among the genes obtained by these methods. The Meg-3 cDNA homologues in species other than humans, such as mouse or rat, can be obtained using similar methods.

Otherwise, the Meg-3 cDNA homologues can be isolated as follows. The cDNA encoding the Meg-3 homologue can be isolated by screening the cDNA library by colony hybridization and plaque hybridization, using the nucleotide sequence of the above-mentioned human Meg-3 cDNA as a probe. The cDNA library can be synthesized by using mRNA isolated from mouse or rat tissues, cultured mesangial cells, and the like as template. A commercially available cDNA library (Funakoshi, etc.) may also be used. PCR that utilizes a degenerative primer, which is designed to be placed before and after ORF, based on the cDNA of human Meg-3 of this invention is another possible method that amplifies the cDNA of homologues.

The human Meg-3 genome can be obtained by screening a genomic library. A genomic library can be synthesized, for example, by preparing a genome from human B lymphoblasts and by inserting, into phage vector EMBL3, DNAs partially digested with Sau3 (Blood, vol 83, No 11, 1994: pp 3126-3131). A clone containing the desired genome can be obtained by performing plaque hybridization (see, Shin Saibou Kougaku Jikken (New Cell Biotechnology Experiment) Protocols, Shujun-sha, pp79-92) for such a genomic library. The entire open reading frame region of Meg-3 cDNA (2202 bp), or each of the exon-intron portions obtained by amplifying the human genomic DNA by PCR method using part of the cDNA as primers can be used as probes. A sequence of 5' UTR of the control region sequence can be determined by 5' RACE method (5'-Full RACE Core Set, following Takara's protocol) using human cultured mesangial cell-derived mRNA or human renal mRNA (purchased from Clontech) as a template.

The gene of the present invention can also be produced by following the standard methods using chemical synthesis of nucleic acids, such as phosphoamidite method (Mattencci, M. D. & Caruthers, M. H. J. Am. Chem. Soc. 103, 3185, 1981), phosphite triester method (Hunkapiller, M. et al., Nature 310, 105, 1984).

An eukaryotic gene often shows polymorphism, like human interferon gene, and one or more amino acids may be replaced by this polymorphism with generally maintaining activities of a protein. In general, activities of proteins can be often maintained even if one or more amino acids are modified. Therefore, gene encoding a protein obtained by using the artificially modified gene encoding an amino acid sequence of SEQ ID NO: 2 is included in this invention as long as the protein possesses the function typical to the gene of the present invention. The present invention includes protein in which an amino acid sequence of SEQ ID NO: 2 is artificially modified as long as it has characteristics of the proteins of the present invention.

The proteins of the present invention comprise the amino acid sequence of SEQ ID NO: 2 or the amino acid sequence in which one or more amino acids are replaced, deleted, added, and/or inserted, and being functionally equivalent to Meg-3 protein of present invention. In this invention, "functionally equivalent" means having biological properties that are the same as Meg-3. The inventor has found the following biological properties in Meg-3, for example.

The Meg-3 may be related to the signal-transducing pathway because of its possession of the PR domain, which binding with the SH3 domain is assumed. SH3 domain is a domain contained in various intracellular signal transduction substances such as the Src family and so on. Further, due to the high possibility (52.2%) of the existence in the cytoplasm of the protein having the amino acid sequence of SEQ ID NO: 2, a great prospect of the Meg-3 to play a part in the signal transduction is indicated.

Furthermore, the Meg-3 has an expression characteristic as follows. At first, one of the characteristics in the human primary cell culture is that the Meg-3 gene is especially highly expressed in mesangial cells. In other primary cell cultures, expression in renal cortical epithelial cell and fibroblast are observed, and slight expression in human endothelial cell of the umbilical vein and smooth muscle cells are observed. Furthermore, when the topography of Meg-3 was detected by Northern blotting, high expression of Meg-3 was observed in the placenta followed by the expression in the pancreas. Additionally, weak expression was observed in kidney, lung and heart, and expression of Meg-3 was hardly observed in liver and skeletal muscles. No expression of Meg-3 could be detected in the brain. The expression of Meg-3 in each of the tissues can be known by performing a Northern blot assay using mRNA prepared from each of the tissues as samples, and using, for example, the nucleotide sequence selected from SEQ ID NO: 1 as a probe.

All proteins that are functionally equivalent with regard to the biological properties mentioned above compose Meg-3 of this invention. Therefore, this invention includes not only human Meg-3, whose structure is specifically elucidated, but also other homologues equivalent in terms of structure or function.

The DNA of the present invention includes DNAs encoding these functionally equivalent proteins. The DNAs encoding these proteins can be cDNA, genomic DNA, or synthetic DNA.

The codons for desired amino acids themselves are well-known, can be optionally selected, and can be determined by following the standard method by, for example, considering the frequency of use of codons in hosts to be used (Grantham, R. et al. Nucleic Acids Res. 9, r43, 1981). Therefore, the present invention includes DNAs appropriately modified by degeneration of codons. It is possible to partially change the codons of these nucleotide sequences by following site-specific mutagenesis methods (Mark, D. F. et al., Proc. Natl. Acad. Sci. U.S.A. 1984, 81: 5662) and such that uses chemically synthesized oligonucleotides encoding the desired modifications as primers.

A DNA hybridizing with a DNA containing the nucleotide sequence of SEQ ID NO: 1 and encoding a protein that typically functions as Meg-3 of the present invention, can be included in the DNA of the present invention. A sequence capable of hybridizing with specific sequences under stringent conditions is thought to have the activities similar to a protein encoded by the specific sequences. Stringent conditions generally indicate the following conditions. That is, hybridization is carried out at 65°C in 4X SSC, and then washing is carried out for one hour at 65°C using 0.1X SSC. The temperature of hybridization and washing, which greatly affect stringency, can be adjusted depending on the melting temperature (Tm). Tm changes, depending on the proportion of constituent bases occupying the base pairs to be hybridized, and by the composition of the hybridization solution (salt concentration, concentrations of formamide and sodium dodecyl sulfate) . Therefore, through experience, one skilled in the art can consider these conditions mentioned above to set appropriate conditions that will yield similar stringency.

The nucleotide sequences of DNA of the present invention, including mutants, can be used for various purposes based on known techniques.

Other prokaryotic or eukaryotic hosts can be transformed by inserting the gene encoding Meg-3 cloned as described above into an appropriate expression vector DNA. Moreover, the gene can be expressed in each host cell by introducing an appropriate promoter and sequences relating to the phenotypic expression into the expression vector. As an expression vector, for example, pET-3 (Studier & Moffatt, J. Mol. Biol. 189, 113, 1986) and such for *E. coli*, pEF-BOS (Nucleic Acids Research 18, 5322, 1990), pSV2-gpt (Mulligan & Berg, Proc. Natl. Acad. Sci. U. S. A. 78, 2072, 1981), and so on for COS cells, and pVY1 (WO89/03874) and such for CHO cells can be used. The target proteins can be expressed as a fusion protein derived from a fusion gene between a target gene and a gene encoding other polypeptide. Such fusion proteins can easily be purified and separated to isolate a desired protein. Histidine tag, c-myc tag, MBP-tag, GST-tag, and the like are known as proteins to be fused. Vectors that can express inserts in which these tags are fused are commercially available.

For example, *Escherichia coli* can be used as prokaryotic host cells in the expression system of the present invention. Saccharomyces *cerevisiae* and such can be used as microbiological host cells among eukaryotic organisms. Examples of mammalian host cells include COS cells, CHO cells, BHK cells, etc. The transformants of the current invention can be cultured under appropriately selected culturing condition suitable for host cells.

As mentioned above, Meg-3 can be produced by culturing the transformants transformed with the gene encoding the target Meg-3, and recovering it from the cells or the culture supernatant. It can be purified into a substantially pure protein. Meg-3, a target protein of the present invention, can be separated and purified by the separation and purification methods commonly used for proteins, and the method is not particularly limited. Meg-3 can be separated and purified by, for example, appropriately selecting and combining various chromatographies.

Besides the methods descried above, the gene of the present invention, the recombinant vector comprising the gene, the transformants carrying the vector, and the production of Meg-3 using gene manipulation can be manipulated by the standard method described in "Molecular Cloning - A Laboratory Manual" (Cold Spring Harbor Laboratory, N. Y.).

In addition, a probe for detecting a Meg-3 gene can be designed based on the nucleotide sequence of SEQ ID NO: 1. Moreover, primers for amplifying DNA and RNA containing these nucleotide sequences can be designed. It is routine for a person skilled in the art to design probes and primers based on a given sequence. An oligonucleotide comprising a designed nucleotide sequence can be chemically synthesized. These oligonucleotides can be used for the hybridization assay of various formats, or for the synthetic reaction of nucleic acids, such as PCR, if appropriately labeled. An oligonucleotide used as a probe or a primer has at least 15 bases, and preferably 25 to 50 bases.

According to the results of the *in situ* hybridization, the present Meg-3 gene expression in the kidney tissue is specifically restricted to the mesangial cell. Thus, the oligonucleotides of the present invention, which specifically hybridizes with the Meg-3 gene, are useful as probes and primers that enable specific detection of the mesangial cell. Due to the fact that the mesangial cell is closely related to the function of the glomerulus, the oligonucleotide of the present invention may serves as a useful tool in pathological analysis of the kidney.

Furthermore, an antisense nucleic acid that may regulate the expression of Meg-3 is provided based on the nucleotide sequence of the gene encoding Meg-3 disclosed in this invention. The antisense nucleic acid of this invention is an important tool for demonstrating the role of Meg-3 in the mesangial cells. It is also useful for regulating diseased conditions caused by accelerated expression of Meg-3. The effects of inhibiting the expression of target genes by antisense nucleic acids are the following. They are inhibition of transcription initiation by triple strand formation, transcription inhibition by hybrid formation with a site that has formed a local open loop structure due to RNA polymerase, transcription inhibition by hybrid formation with RNA that is being synthesized, splicing inhibition by hybrid formation at the joint between intron and exon, splicing inhibition by hybrid formation with the spliceosome forming region, inhibition of mRNA transition from the nucleus to the cytoplasm by hybrid formation with mRNA, splicing inhibition by hybrid formation with the capping region and the poly(A) attached region, inhibition of translation initiation by hybrid formation with the translation initiation factor binding region, translation inhibition. by hybrid formation with the ribosome binding region near the initiation codon, interception of protein chain elongation by hybrid formation with the translation region of mRNA and polysome binding region, and inhibition of gene expression by hybrid formation with the nucleic acid-protein interacting region, and so on. These inhibit the expression of the target gene by inhibiting the transcription, splicing, or translation processes (Hirashima and Inoue, Shin Seikagaku Jikken Koza 2 Kakusan IV Idenshi no Fukusei to Hatsugen (New Biochemistry Laboratory Experiment 2 Nucleic Acids IV Replication and Expression of Genes) , Japanese Biochemical Society Edition, Tokyo Kagaku Dojin, pp. 319-347, 1993).

The antisense sequence used in this invention may inhibit the expression of the target genes by any one of the effects mentioned above. In one embodiment, if an antisense sequence complementary to the non-translational region located near the 5' end of mRNA of the gene is designed, it may be effective for inhibiting the translation of the gene. However, sequences complementary to the coding region or to the 3'end non-translational region may also be used. In this manner, the antisense DNA utilized in this invention includes DNA containing the antisense sequence of not only the translational region of the gene, but also the sequence of the non-translational region. The antisense DNA to be utilized is inserted downstream of the appropriate promoter, and preferably, a sequence including a transcription-terminating signal is inserted to the 3' end. DNA prepared in this manner can be transformed into the desired host by known methods. Preferably, the antisense DNA sequence should contain a sequence that is complementary to the endogenous gene (or its homologous genes) of the host to be transformed or its portion. However, as long as gene expression is inhibited effectively, complete complementarity is not necessary.

The RNA that is transcribed using antisense DNA as template is designed to have preferably 90% and most preferably 95% complementarity to the transcription product of the target gene. The length of the antisense DNA used for effectively inhibiting the expression of target genes is at least 15 nucleotides or more, preferably 100 nucleotides or more, and more preferably 500 nucleotides or more. Usually, the length of the antisense RNA used is shorter than 2.5 kb.

A promoter region and an enhancer region of Meg-3 gene existing in genome can be obtained based on the cDNA nucleotide sequence of Meg-3 of the present invention. Specifically, these control regions can be obtained by the same method as described in unexamined published Japanese patent application (JP-A) No. Hei 6-181767; The Journal of Immunology, 1995, 155, 2477-2486; Proc. Natl. Acad. Sci. USA, 1995, 92, 3561-3565; etc. Herein, a promoter region means DNA region existing upstream of a transcription initiation site to control the expression of a gene, and an enhancer region means DNA region existing in an intron or 3'UTR to control expression of a gene.

Specifically, a promoter region can be obtained, for example, by the following method.
1) A promoter region of Meg-3 is cloned from a human genomic library using 5' end site of cDNA of Meg-3 as a probe.
2) Meg-3 gene is digested with restriction enzyme to obtain a DNA comprising the promoter region at the upstream region (2 to 5 kbp) containing a translation initiation codon of Meg-3 gene and determine the nucleotide sequence. The transcription initiation site (+1) is determined using poly(A)⁺RNA prepared from human mesangial cells as a template, by the primer elongation method using primer DNA selected from cDNA sequence at 5' end site of Meg-3 gene. A site possibly comprising the promoter activity is predicted by searching transcription factor binding sequence from the nucleotide sequence.
3) The DNA fragment excluding the coding region of Meg-3 gene from the DNA obtained in 2) is subcloned in a plasmid, and a chloramphenicol acetyl transferase (CAT) gene or a luciferase gene is ligated as a reporter gene at 2 to 5 kbp downstream of the DNA fragment to construct a reporter plasmid. Similarly, DNA fragments corresponding to various sites upstream of Meg-3 gene, in which 5' and 3' end sites are stepwise removed, are prepared by digestion with restriction enzymes or by PCR to include possible promoter regions. The CAT gene or the luciferase gene is ligated as a reporter gene at downstream of these DNA fragments to construct a reporter plasmid.
4) A promoter region upstream of Meg-3 gene is obtained by measuring CAT or luciferase activity in animal cells transformed with the reporter plasmid prepared in 3).

A 3' UTR and an enhancer region in introns can be obtained by cloning genomic genes of human Meg-3 from a human genomic library using Meg-3 cDNA as a probe in the same manner as described above for the promoter.

Transcription factors controlling the expression of Meg-3 gene can be obtained by the known methods, for example, those described in "Shin Saibou Kougaku Jikken (New Cell Biotechnology Experiment) Protocols, Shujun-sha," "Biomanual series 5 Tensha Inshi Kenkyu-hou (studies on transcription factors), Yodo-sha," "DNA & Cell Biology, 13, 731-742, 1994," such as affinity chromatography, South-western method, footprinting method, gel shift method, or one-hybrid method. Herein, a transcription factor means a factor controlling the transcription of Meg-3 gene, including a transcription initiation factor that induces the transcription initiation reaction and a transcription control factor that up- or down-regulates transcription.

Affinity chromatography can be performed by applying a nucleic extract to an affinity column in which promoter and enhancer regions obtained above are immobilized on Sepharose or latex beads, washing the column, eluting the binding transcription factor using a DNA comprising the same sequence as that immobilized in the column, and recovering the transcription factor controlling the expression of Meg-3 gene.

In case where the South-Western method is employed, the expression product of the cDNA inserted in the *E.coli* expression vector (e.g. λgt11) is screened as per the tagged-probe. For example, the cDNA to be screened is expressed as a fusion protein with the β-galactosidase, and is adsorbed to the nitrocellulose filter. Then, the transcription factor, which regulates the Meg-3 gene expression, can be obtained by selecting those phages that synthesize the fusion protein with a binding activity using radioisotope tagged DNA fragments of the promoter region or enhancer region as the probe.

The gel shift method is based on the phenomenon that electrophoretic mobility on a polyacrylamide gel of DNA changes when it is bound to a protein. DNA fragments of the promoter region and enhancer region are used as probes and upon mixing with samples containing transcription factors (for example, nuclear protein extract), they are analyzed by electrophoresis under low ionic strength. The binding of transcription factors is detected as a band with mobility different from that of free DNA. The gel shift method allows separation of transcription factors from a mixture of proteins with high sensitivity.

Upon further analysis by the footprint method, the DNA-transcription factor complex obtained by the gel shift method allows determination of the transcription factor-binding site. The footprint method utilizes the phenomenon that when a protein binds to DNA, it is protected from DNase I digestion. That is, DNA of the promoter region and enhancer region labeled at the end with ³²P is partially digested by DNase I in the presence of transcription factors, and then separated by degenerate polyacrylamide gel used for determining nucleotide sequences. Comparison to the result when the same treatment is carried out in the absence of a transcription factor shows the disappearance of a band due to transcription factor binding, and therefore, estimation of the binding region becomes possible.

The present invention also provides an antibody recognizing Meg-3. The antibody of the present invention includes, for example, an antibody against the protein comprising the amino acid sequence of SEQ ID NO: 2. An antibody (for example, a polyclonal antibody, a monoclonal antibody) or an antiserum against Meg-3 or a partial peptide of Meg-3 of the present invention can be produced by a known method for producing an antibody and antiserum, using Meg-3 of the present invention, a partial peptide of Meg-3 of the present invention, or a fusion protein such as c-myc-(His)₆-Tag-Meg-3 or MBP-Meg-3 of the present invention as a antigen.

The Meg-3 of the present invention or a partial peptide of Meg-3 of the present invention is administered with well-known carrier or diluent to a warm-blooded animal at the site capable of producing an antibody. To enhance the antibody productivity, the complete Freund's adjuvant or incomplete Freund's adjuvant can be administered together with the antigen. Immunization is performed every one to six weeks, a total of about 2 to 10 times, in general. Warm-blooded animals to be used are, for example, a monkey, rabbit, dog, guinea pig, mouse, rat, sheep, goat, and chicken, and preferably a mouse and rat.

Monoclonal antibody-producing cells can be prepared by selecting immunized warm-blooded animals, such as mice, in which an antibody titer is detected, obtaining spleen or lymph node from the animals 2 to 5 days after the final immunization, and fusing the antibody producing cells contained in these tissues with myeloma cells to obtain monoclonal antibody-producing hybridoma. The antibody titer in antiserum can be measured by, for example, reacting the labeled Meg-3 described below with antiserum, and measuring an activity of the label binding to the antibody. Cell fusion can be performed by a known method, for example, the method of Kohler and Milstein (Nature, 256, 495, 1975). Polyethylene glycol (PEG) , Sendai virus, and such can be used as a fusion enhancer, and PEG is preferable.

Examples of myeloma cells include X-63Ag8, NS-1, P3U1, SP2/0, AP-1, and such, and X-63Ag8 is preferably used. The ratio of the number of antibody-producing cells (splenic cells) to that of myeloma cells is 1:20 to 20:1. Cells can be fused efficiently by adding PEG (preferably PEG1000 to PEG6000) at the concentration of about 10 to 80%, and incubating for 1 to 10 min at 20 to 40°C, preferably at 30 to 37°C. Anti-Meg-3 antibody-producing hybridoma can be screened by various methods, for example, the method in which the hybridoma culture supernatant is added to a solid phase (for example, a microplate) on which Meg-3 antigen is adsorbed directly or with a carrier, and anti-immunoglobulin antibody labeled with a radioactive substance, an enzyme, and such (When cells used for cell fusion are derived from a mouse, anti-mouse immunoglobulin antibody is used.) or protein A is added thereto, and anti-Meg-3 monoclonal antibody binding to the solid phase is detected, the method in which the hybridoma culture supernatant is added to a solid phase on which anti-immunoglobulin antibody or protein A is adsorbed, and Meg-3 labeled with a radioactive substance, an enzyme, and such is added thereto, and anti-Meg-3 monoclonal antibody binding to the solid phase is detected.

Anti-Meg-3 monoclonal antibody can be selected and cloned by known methods or modified methods thereof using usually a culture medium for animal cells supplemented with HAT (hypoxanthine, aminopterin, and thymidine). Any medium for selection, cloning, and culturing can be used as long as hybridoma can grow therein. For example, RPMI 1640 medium (Dainippon Pharmaceutical) containing 1 to 20%, preferably 10 to 20% of fetal bovine serum, GIT medium (Wako Pure Chemicals) containing 1 to 10% fetal bovine serum, or serum-free medium for hybridoma culturing (SFM-101, Nissui Pharmaceutical) can be used. Incubation temperature is generally 20 to 40°C, preferably about 37°C. Incubation time is generally 5 days to 3 weeks and preferably 1 to 2 weeks. Incubation is performed under the 5% carbon dioxide gas in general. The antibody titer of the hybridoma culture supernatant can be determined in the same manner as described above for the measurement of anti-Meg-3 antibody titer in the antiserum. Cloning can be generally conducted by known methods, for example, semisolid agar method, or limiting dilution method. A cloned hybridoma is cultured preferably in a serum-free medium, thereby producing an optimal amount of an antibody in the supernatant. A target monoclonal antibody can be obtained in ascites.

A monoclonal antibody of the present invention does not crossreact with other proteins other than Meg-3 by selecting those capable of recognizing epitopes specific to Meg-3. In general, an epitope specific to a protein is composed of at least 7 or more continuous amino acid residues, preferably 10 to 20 amino acids in an amino acid sequence of the protein. Therefore, a monoclonal antibody recognizing an epitope composed of peptides having an amino acid sequence selected from the amino acid sequence of SEQ ID NO: 2 and composed of at least 7 continuous amino acid residues can be the monoclonal antibody specific to Meg-3 of the present invention.

An anti-Meg-3 monoclonal antibody can be separated and purified by the separation and purification method of immunoglobulin commonly used for the separation and purification of polyclonal antibodies. The known purification methods include, for example, salting out, alcohol precipitation, isoelectric point precipitation, electrophoresis, adsorption and desorption method by ion exchanger (for example, DEAE), ultra centrifugation, gel filtration, or specific purification method whereby antibody is exclusively collected by, for example, an antigen binding solid phase or active adsorbent, such as Protein A or Protein G, and the binding is dissociated to obtain the antibody.

Monoclonal antibodies and polyclonal antibodies recognizing Meg-3 of the present invention, obtained in such a manner, can be used for the diagnosis and treatment for diseases relating to mesangial cells. Examples of a method for measuring Meg-3 with these antibodies include an sandwich assay comprising reacting Meg-3 with an antibody binding to an insoluble carrier and a labeled antibody and detecting Meg-3 in the sandwiched complex produced by the reaction, or a competition method comprising competitively reacting labeled human Meg-3 and human Meg-3 in a sample with an antibody to measure human Meg-3 in a samples based on labeled antigen amount reacted with the antibody.

The measurement of human Meg-3 by the sandwich method is conducted by, for example, the 2 step method in which an immobilized antibody is reacted with Meg-3, unreacted materials are completely removed by washing, and a labeled antibody is added to form a complex of the immobilized antibody Meg-3 the labeled antibody, or one step method in which the immobilized antibody, the labeled antibody, and Meg-3 are mixed at the same time.

Examples of an insoluble carrier used for the measurement include, for example, polystyrene, polyethylene, polypropylene, polyvinyl chloride, polyester, polyacrylate, nylon, polyacetal, synthetic resin such as fluoride resin, etc., polysaccharides such as cellulose, agarose, and such, glass, metals, etc. The form of an insoluble carrier can be varied and includes tray, spheroid, particle, fiber, stick, board, container, cell, test tube, etc. The antibody-adsorbed carrier should be stored at a cool place in the presence of appropriate preservatives, such as sodium azide.

Antibodies can be immobilized by known chemical binding or physical adsorption methods. Chemical binding methods include, for example, a method using glutaraldehyde, the maleimide method using N-succinimidyl-4-(N-maleimidomethyl)cyclohexane-1-carboxylate, N-succinimidyl-2-maleimidoacetate, and such, and the carbodiimide method using 1-ethyl-3-(3-dimethylaminopropyl)carbodiimide hydrochloride, etc. In addition, the maleimidobenzoyl-N-hydroxysuccinimidoester method, the N-succimidyl-3-(2-pyridyldithio)propionate method, the bisdiazolated benzidine method, and dipalmityllysine method. Alternatively, the complex produced by reacting two different antibodies against a substance to be detected and an epitope is captured with the third antibody immobilized by the above method.

Any label useful for immunoassay can be used without being limited. Specifically, enzymes, fluorescent substances, luminescent substances, radioactive substances, metal chelates, and such, can be used. Preferable labeling enzymes are, for example, peroxidase, alkaline phosphatase, β-D-galactosidase, malate dehydrogenase, *Staphylococcus* nuclease, delta-5-steroid isomerase, α-glycerol phosphate dehydrogenase, triosephosphate isomerase, horseradish peroxidase, asparaginase, glucose oxidase, ribonuclease, urease, catalase, glucose-6-phosphate dehydrogenase, glucoamylase, and acetylcholine esterase, etc. Preferable fluorescent substances include, for example, fluorescein isothiocyanate, phycobiliprotein, rhodamine, phycoerythrin, phycocyanin, allophycocyanin, and orthophthalaldehyde. Preferable luminescent substances include, for example, isoluminol, lucigenin, luminol, aromatic acridiniumester, imidazole, acridinium salt and its modified ester, luciferin, luciferase, and aequorine. Preferable radioactive substances include, for example, ¹²⁵I, ¹²⁷I, ¹³¹I, ¹⁴C, ³H, ³²P, ³⁵S, etc.

The method for binding the above labels is known. Specifically, direct and indirect labeling can be used. The common direct labeling is the method in which an antibody or an antibody fragment is chemically covalent-bound with a label using a cross linking agent. Crosslinking agents include N,N'-orthophenylenedimaleimide, 4-(N-maleimidomethyl) cyclohexanoate N-succinimide ester, 6-maleimidohexanoate N-succinimide ester, 4,4'-dithiopyridine, and other known crosslinking agents. The crosslinking agent can be reacted with enzymes and antibodies by the known methods depending on the characteristics of the crosslinking agent. An example of the indirect labeling method comprises binding an antibody to a low molecular weight hapten such as biotin, dinitrophenyl, pyridoxal, or fluorescamine, and indirectly labeling the antibody with the binding partner to the hapten. Avidin and streptoavidin can be used as a recognition ligand for biotin, whereas dinitrophenyl, pyridoxal, or fluorescamine are labeled with antibodies recognizing these haptens. Horseradish peroxidase can be used as an enzyme for labeling antibodies. This enzyme is useful because it can react with many substrates and be easily bound to antibodies by the periodate method. Occasionally, as an antibody, their fragments, for example, Fab', Fab, F(ab')₂ are used. Both polyclonal and monoclonal antibodies can be labeled with an enzyme by the same method. Enzyme-labeled antibodies obtained using the above crosslinking agent can be purified by the known methods such as affinity chromatography, and such, to serve in a more sensitive immunoassay system. Purified enzyme-labeled antibodies are stored with a preservative such as thimerosal and a stabilizer such as glycerol. Labeled antibodies can be lyophilized and stored in the cool and dark place for a long time.

When a label is an enzyme, its substrate and, if necessary, a coloring agent is used for measuring its activity. When peroxidase is used as an enzyme, H₂O₂ is used as a substrate solution and 2,2'-azino-di-[3-ethylbenzothiazolinesulfonic -acid] ammonium salt (ABTS), 5-aminosalicylic acid, orthtophenylenediamine, 4-aminoantipyrine, or 3,3',5,5'-tetramethylbenzidine, and such, is used as a coloring agent. When alkaline phosphatase is used as an enzyme, orthonitrophenylphosphate, paranitrophenylphosphate, and such, can be used as substrates. When β-D-galactosidase is used as an enzyme, fluorescein-di-(β-D-galactopyranoside), 4-methylumbelliferyl-β-D -galactopyranoside, and such, can be used as substrates. The present invention also includes an immunoassay reagent for Meg-3, comprising labeled or immobilized monoclonal or polyclonal antibodies, and further includes a kit comprising this reagent and an indicator for detection label and a control sample, etc.

Any biological samples such as body fluid such as blood plasma, serum, blood, urine, tissue fluid, or cerebrospinal fluid and such can be used as samples for measuring the Meg-3 of the present invention as long as they contain Meg-3 or its precursor or a fragment.

In addition, the present invention relates to a transgenic nonhuman vertebrate in which the expression level of Meg-3 gene is altered. Herein, Meg-3 gene includes cDNA, genomic DNA, or synthetic DNA encoding Meg-3. Gene expression includes both steps of transcription and translation. Transgenic animals of the present invention are useful for investigating function and expression control of Meg-3, clarifying mechanisms of development of diseases relating to human mesangial cells, and developing disease model animals used for screening and testing safety of pharmaceuticals.

In the present invention, Meg-3 gene can be modified so as to artificially increase or decrease its expression level compared with the original gene by introducing mutation such as deletion, substitution, insertion, and such, in a part of some important sites (enhancer, promoter, intron, etc.) which control the normal expression of Meg-3 gene. Such modification alters transcription of Meg-3 gene. On the other hand, translation to proteins can be modified by deleting a part of an exon, or replacing a certain codon with a stop codon by introducing point mutation into coding regions. Such mutation can be introduced by the known methods for obtaining transgenic animals.

Transgenic animals means, in a narrow sense, animals into reproductive cells of which an exogenous gene is artificially introduced by genetic recombination, and in a broad sense, animals into chromosome of which an exogenous gene is stably introduced during an early developmental stage, the gene can be transmitted to the offspring as genotype, including antisense transgenic animals in which the function of a specific gene is inhibited by antisense RNA, animals in which a specific gene is knocked out by using embryonic stem cells (ES cells), and animals into which point mutation DNA is introduced. Transgenic animals used herein include all vertebrates except for human.

Transgenic animals can be prepared by the method comprising mixing a gene with an egg and treating the mixture with calcium phosphate, the microinjection method whereby a gene is directly injected into a nucleus in pronuclear egg by a micropipette under the phase contrast microscope (microinjection method, U.S. Patent No. 4,873,191), and the method using embryo stem cells (ES cells). Other methods include, for example, the method in which a gene is inserted into a retrovirus vector to infect an egg and the sperm vector method in which a gene is introduced into an egg through sperm, etc. The sperm vector method is a genetic recombination method for introducing an exogenous gene by attaching an exogenous gene into sperm or incorporating an exogenous gene into sperm cells by electroporation, etc. and fertilizing an egg (M. Lavitranoet et al., Cell, 57, 717, 1989).

In vivo site-specific genetic recombination such as cre/loxP recombinase system of bacteriophage P1, FLP recombinase system of *Saccharomyces cerevisiae*, and such, can be used. The method for introducing a transgene of a target protein into nonhuman animals using retrovirus has been reported.

Methods for preparing transgenic animals by microinjection are performed, for example, in the following manner. A transgene basically composed of a promoter regulating expression, a gene encoding a specific protein, and poly (A) signal is provided. Expression pattern and level for all lineages should be confirmed since the expression pattern and level of a specific molecule depend on the promoter activity, and prepared transgenic animals vary among lineages depending on the number of copies and introduction site on chromosomes of an introduced transgene. A sequence of introns to be spliced at upstream of poly (A) signal may be introduced when the expression level is known to vary depending on noncoding region and splicing. It is important to use a gene as pure as possible for introducing into a fertilized egg. An animal to be used includes a mouse for collecting fertilized eggs (5 to 6 weeks old) , male mouse for crossing, pseudopregnant female mouse, vasoligated male mouse, etc.

To efficiently obtain fertilized eggs, ovulation can be induced by gonadotropin, etc. A fertilized egg is collected, and a gene is injected into a male pronucleus of the egg by microinjection using an injection pipette. Animals for returning the treated eggs into an oviduct are prepared (pseudopregnant female mice, etc.), and about 10 to 15 eggs are transplanted per each individual. Introduction of the transgene into a new-born mouse is confirmed by extracting genomic DNA from the tip of the tail and detecting the transgene by Southern hybridization or PCR methods, or by the positive cloning method in which a marker gene that is activated only upon homologous recombination is inserted. Expression of the transgene can be confirmed by detecting a transgene-derived transcript by Northern hybridization or RT-PCR methods. Detection by Western blotting method is also possible using an antibody specific to a protein.

A knockout mouse of the present invention is prepared so as to lose the function of mouse Meg-3 gene. A knockout mouse means a transgenic mouse in which a certain gene is destroyed by homologous recombination technology to eliminate its function. A knockout mouse can be prepared by conducting homologous recombination using ES cells and selecting ES cells in which one allele is modified and destroyed. For example, genetically manipulated ES cells are injected into a blastocyst or an 8-cell embryo of a fertilized egg to obtain a chimeric mouse having both cells derived from ES cells and from embryo. A heterozygous mouse in which all of one allele is modified and destroyed can be prepared by crossing a chimeric mouse (chimera means an individual composed of somatic cells derived from two or more fertilized eggs) and a normal mouse. Crossing of heterozygous mice with each other can produce homozygous mice. A transgenic animal of the present invention includes both heterozygotes and homozygotes.

Homologous recombination means the recombination occurring between two genes whose nucleotide sequences are the same or extremely similar through mechanism of genetic recombination. Cells with homologous recombination can be selected by PCR. Homologous recombination can be confirmed by performing PCR using as primers sequences of a part of a gene to be inserted and a part of a chromosomal region into which the gene is expectedly inserted and detecting cells producing amplified products. Homologous recombination in the genes expressed in ES cells can be easily screened by known methods or their modified methods, for example, by binding neomycin resistant gene to the introduced gene to make the cells neomycin resistant after the introduction.

### Brief Description of the Drawings

Figure 1 is a photograph showing the results of the Western blot analysis with anti-Meg-3 antibody. Each lane corresponds to the following antigen;
   Lane 1: MBP
   Lane 2: MBP-Meg-3
   Lane 3: Luciferase protein expressed by *in vitro* transcription and translation using rabbit reticulocyte (Promega)
   Lane 4: Meg-3 tagged with c-myc to the 3'-terminus
Figure 2 is a micrograph showing the result of speculum by confocal laser microscope of the CHO cell, in which 3'-terminus c-myc tagged Meg-3 is overexpressed. The nucleus is stained blue in the center, and Meg-3 is localized around it and is stained green by fluorescence.

### Best Mode for Carrying out the Invention

The present invention is illustrated in detail below with references to examples, but is not to be construed as being limited thereto.

### Example 1: Primary culture of human mesangial cells

Human glomerular renal mesangial cells were isolated from the normal human kidney excised from a 58 year-old male. Renal cortex was separated under the sterilized condition, minced, and passed through several sieves. Pore diameters of the used sieves were decreased stepwise, and the trapped glomerulus by the sieve at the pore diameter of 75 to 200 µm was washed and incubated with 100 µg/ml collagenase (Washington Biochemical) at 37°C for 20 min. After washing, the glomerulus was resuspended in medium 199 (Gibco BRL, Gaithersburg, MD) containing 25 mM Hepes, 10% Nu-serum (Collaborative Biomedical Products, Bedford, MA), and antibiotics (10 µg/ml of penicillin, streptomycin, and fungizone), and incubated in the 5% CO₂ incubator. At the third passage, mesangial cells were identified based on a series of criteria such as typical morphological characteristics, resistance to trypsin, puromycin, and D-valine, positiveness against immunostaining of actin (Zymed Laboratories, San Francisco, CA), anti-very late antigen (VLA)-1, 3,5 (Immunotech), and negativeness against immunostaining of VIII factor (Dako, CA).

### Example 2: Isolation of mRNA from human cultured mesangial cells

At the sixth passage, total RNA was isolated from human mesangial cells using guanidine isothiocyanate (GTC) method. The confluent culture of the mesangial cells in the medium containing serum of the cells of Example 1 was washed with phosphate buffer saline (PBS), and dissolved in 5.5 mM GTC solution. DNA was removed by passing through an 18-gauge needle. Nuclei and other cell debris were precipitated by centrifugation at 5,000X g for 90 sec. Supernatant was carefully loaded on the layer of cesium trifluoroacetate (CsTFA) and centrifuged at 125,000X g at 15°C for 24 hours. RNA pellet was dissolved in TE buffer. Poly(A)⁺ RNA was isolated using oligo dT cellulose column (Pharmacia).

### Example 3: Construction of 3'-directed cDNA library

cDNA was synthesized using the vector primer based on pUC19 (Norrander J. et al., Gene, 26, 101-106, 1983) with poly (A)⁺ RNA as a template. This vector primer DNA comprised the HincII end and the PstI end with a T tale, and dam-methylated at the MboI site (GATC). After synthesizing the second strand, the cDNA sequence and the single BamHI site in LacZ gene of the vector were digested with MboI and BamHI, respectively, and circularization and ligation were conducted at the low DNA concentration. A portion of the ligation mixture was transformed to *E. coli.* The obtained transformants were randomly selected and individually dissolved by simply heating. The inserted sequence of cDNA was amplified by the paired PCR using primers (5'-TGTAAAACGACGGCCAGT-3'/SEQ ID NO: 3 and 5'-ACCATGATTACGCCAAGCTTG-3'/SEQ ID NO: 4) flanking the pUC19 cloning site. The obtained short double stranded DNA was used for the cycle sequence determination reaction and analyzed by an automatic sequencer.

### Example 4: Isolation of genes expressed specifically in mesangial cells

In order to identify genes expressed specifically in mesangial cells, the present inventor conducted large scale DNA sequencing and data processing by computers. Transcripts in the various different cells and organs could be simultaneously compared (Y. Yasuda et al., Kidney International 53:154-158, 1998; K. Matsubara et al., Gene. 135, 265 (1993); K. Okubo et al., Nat. Gen. 2, 173 (1992)). Large scale DNA sequencing of the 3'-domain cDNA library of human cultured mesangial cells was conducted, and randomly selected 1836 clones were sequenced for their partial sequences. The sequence homology among clones was mutually compared, and further compared with that in DNA data bank GenBank using FASTA program. mRNA from various organs and cells were analyzed using dot blot to select clones expressed specifically in mesangial cells. As a result, some clones detected at extremely high frequency in the mesangial cell cDNA library were obtained.

### Example 5: Screening of human mesangial cell λZIPLox cDNA library

From the complete mRNA prepared according to Example 2, the λZIPLox cDNA library was synthesized using oligo (dT) primer and random primer. Commercially available λZIPLox (Gibco BRL, λZIPLox *EcoRI* Arms) was used to synthesize this library. This λZIPLox cDNA library was screened for a specific clone that was detected particularly frequently in the mesangial cell cDNA library, obtained in Example 4, by using its insert as a probe. The nucleotide sequence of the inserted gene segment was determined for the positive clone by the dideoxy termination method.

Sequence determination results showed that the deduced location of initiation codon ATG was consistent with that of the consensus sequence and gave the longest open reading frame ("the first ATG rule" is satisfied). The Meg-3 cDNA nucleotide sequence is described in SEQ ID NO: 1, and the deduced amino acid sequence of Meg-3 is shown in SEQ ID NO: 2.

### Example 6: Functional analysis of a mesangium-specific gene (1)

The amino acid homology search performed by the program FASTA using the SwissProt database confirmed that there is no known amino acid sequence with high homology with the Meg-3 protein, and that Meg-3 is a novel protein.
Next, a motif search was carried out on the Meg-3 amino acid sequence. PSORT WWW Server (http://psort.nibb.ac.jp:8800/) was used for the search. As a result, an amino acid sequence that closely resembles many proteins called proline rich protein was found at regions following after the 500th amino acid counted from the N-terminus. The region comprising the 81 amino acid residues from 621st amino acid to 701st amino acid is especially abound in proline (27.2%), and possess at two points the amino acid sequence (xPESPPPAxP), which resembles the amino acid sequence (xPxxPPPFxP) of the proline rich peptide (PR peptide) that binds to the SH3 (Src homology 3) domain. In addition, existence of phosphorylation motifs for phosphorylation enzymes as follows was confirmed. Also, two N-myristoylation sites were found. The predicted percentage of localization in the cytoplasm was 52.2%. These findings strongly indicate that Meg-3 is a signal-transducing factor.
casein kinase II phosphorylation site: 14
protein kinase C phosphorylation site: 9
tyrosine kinase phosphorylation site: 1

Furthermore, based on these facts, it was confirmed that the above-predicted amino acid sequence with 733 amino acid residues corresponds to the amino acid sequence of Meg-3. The calculated molecular weight and the theoretical pI of the protein with this amino acid sequence are about 83 kDa and 5.72, respectively.

### Example 7: Functional analysis of Meg-3 (2) - tissue distribution

Northern blot analysis of Meg-3 was performed as described in the following. The positive clone insert of the 3' directed cDNA library (Example 3) was labeled with RI by random DNA labeling and was used as a probe. Poly (A) ⁺RNA (2 µg) isolated from the below-mentioned cells to be used as samples was separated on a 1% agarose gel containing 2.2 M formamide and was transferred onto a nitrocellulose filter. The filter was hybridized in Rapid Hyb solution (Amersham, Arlington Heights, IL). After hybridization, it was washed with final stringency of 0.1X SSPE/0.1% SDS at 60°C.

Samples for Northern blot analyses of multiple human primary culture cells and tissues, and for Northern blot analysis of human cancer cell lines were purchased from Clontech (Palo Alto, CA). As samples of primary culture cells, 2 µg of poly (A) ⁺RNA from primary culture cells of human mesangial cells, human dermal fibroblast cells, human renal cortical epithelial cells, human endothelial cells of the umbilical vein, and human smooth muscle cells were used. For Northern blot analysis of human cancer cell lines, 2 µg of poly (A) ⁺RNA derived from promyelocytic leukemia HL-60, HeLa cells S3, chronic myeloid'leukemia K-562, lymphoblastic leukemia MOLT-4, Burkitt's lymphoma Raji, adenocarcinoma of the large intestine SW480, lung cancer A549, and melanoma G361 were used as samples. For Northern blot analysis of the tissues, 2 µg of poly (A) ⁺RNA derived from the heart, brain, placenta, lung, liver, skeletal muscles, kidney and pancreas were used as samples. Hybridization and washing were carried out as described above. The results are as indicated in Tables 1 to 3.

**Table 1**

| | |
|---|---|
| Primary culture cells | |
| Human mesangial cells | + + + |
| Human dermal fibroblast cells | + + |
| Human renal cortical epithelial cells | + + |
| Human endothelial cells of the umbilical vein | ± |
| Human smooth muscle cells | ± |

**Table 2**

| | |
|---|---|
| Human cancer cell lines | |
| Promyelocytic leukemia HL-60 | - |
| HeLa cells S3 | + + + |
| Chronic myeloid leukemia K-562 | + |
| Lympphoblastic leukemia MOLT-4 | - |
| Burkitt's lymphoma Raji | - |
| Adenocarcinoma of the large intestine SW480 | + + + |
| Lung cancer A549 | + + |
| Melanoma G361 | + |

**Table 3**

| | |
|---|---|
| Human tissues | |
| Heart | + |
| Brain | - |
| Placenta | + + + |
| Lungs | + |
| Liver | ± |
| Skeletal muscle | ± |
| Kidney | + |
| Pancreas . | + + |

In the Northern blot analysis using Meg-3 cDNA as a probe, a single transcription product (approximately 4.0 kb) was found in the cultured mesangial cells. One of the characteristics in the human primary cell culture was that the Meg-3 gene is especially highly expressed in mesangial cells. In other primary cell cultures, expression in renal cortical epithelial cell and dermal fibroblast are observed, and slight expression in human endothelial cell of the umbilical vein and smooth muscle cells was observed. Furthermore, when those in tissues are compared, high expression of Meg-3 was observed in the human placenta followed by the expression in the pancreas. Further, expression in tissues like heart, lung, and kidney were observed. Expression in the liver and skeletal muscle was weak, and no expression could not be detected in the brain. No noticeable expression was observed in cultured cancer cell lines except the strong expression in HeLa cell S3 and adenocarcinoma of the large intestine SW480, and expression in the lung cancer A549.

### Example 8: Functional analysis of a Meg-3 (3) -in situ hybridization

Meg-3 mRNA expression was evaluated in a normal human renal tissue by in situ hybridization (abbreviated as ISH hereinafter). ISH was conducted following known methods (Kidney Int. 52:111 (1997)). Nucleotide sequence corresponding to position 404 to 433 of the human Meg-3 cDNA (SEQ ID NO: 5) was used as the probe. Meg-3 transcription products were localized in the mesangial cell in the glomerulus. Pretreatment of the tissue with the RNase before hybridization to assess the specificity of the detected signal resulted in elimination of most signals detected by Meg-3 probes. Furthermore, according to the competitive examination using 100-fold excessive untagged oligonucleotides with the same sequence or unrelated sequence, the signal from the Meg-3 probe disappeared by the use of the oligonucleotide with the same nucleotide sequence, whereas it remained by the unrelated oligonucleotide. These results qualified that the Meg-3 gene having the nucleotide sequence described in SEQ ID NO: 1 is specifically expressed in the mesangial cell.

### Example 9: Expression of the Meg-3 protein

PCR was conducted using 1.0 µl human cultivated mesangial cell poly (A)⁺ RNA (0.5 µg/µl) as the template and following primers designed to encode the translational region, to obtain the gene comprising the translational region of Meg-3. The primers used were: (1) a primer including the initiation codon and the restriction enzyme EcoRI recognition site added to the 5' terminus (5'-CGGAATTCATGGGGTGGATGGG-3'/ SEQ ID NO: 6), and (2) the primer including the stop codon and the EcoRI recognition site (5'-GCGAATTCTAGAACTCAGTCTGCACCCCTGC-3' / SEQ ID NO: 7). The reaction mixture contained 5 µl 10X Ex Taq buffer, 8 µl dNTP mixture (2.5 mM), 0.5 µl PCR primer (SEQ ID NO: 6, 20 pmol/µl), 0.5 µl primary PCR A1 primer (SEQ ID NO: 7, 20 pmol/µl), and 0.5 µl TaKaRa Ex TaqTM (10 U/µl) and was filled up to a total volume of 50 µl with sterilized water. The mixture was set in the "Takara PCR Thermal Cycler", reacted at 94°C for 1 minute, 60°C for 2 minutes and 72°C for 2 minutes, and that for 30 cycles. The band was confirmed by the 0.75% agarose gel electrophoresis method. Using 1 µl of the reaction solution, the DNA was subcloned by means of "Original TA Cloning Kit" (Invitrogen), arid the obtained plasmid was named meg3/pCR2. The plasmid was cleaved with EcoRI, ligated with EcoRI digested maltose binding protein fusion protein expression vector pMAL-c2 (New England Biolab) using T4 ligase, and the obtained vector was transformed into the *E.coli* JM109. After 18 hours, ampicillin resistant strains were seeded in 3 ml of LB medium and incubated for 18 hours. Plasmids were extracted as the expression vector pMALc2/meg3 according to the mini-prep method, and were confirmed by the use of restriction enzymes.

The *E.coli* XL1-Blue transformed with pMALc2/meg3 was incubated for 18 hours at 37°C with shaking in 10 ml LB medium supplemented with ampicillin to a concentration of 100 µg/ml. The incubation solution was added to 1 L of Rich medium (containing 10 g triptone, 5 g yeast extract, 5 g NaCl, 2 g glucose in 1 L, and ampicillin added to a concentration of 100 µg/ml) and was further incubated at 37°C with shaking. After the measurement of the turbidimeter reached an OD of about 0.8 (A600), 3 ml of 0.1 M IPTG (1.41 g isopropyl-β-D-thiogalactoside dissolved in 50 ml water) was added, and the mixture was further incubated at 37°C with shaking. Harvested the cell body by centrifugation (4,000 g X 20 minutes) after 2 hours, 50 ml of lysis buffer (10 mM Na₂HPO₄, 30 mM NaCl, 0.25% Tween20, pH 7.0) was added. Suspended the cell bodies well in the buffer, after freezing 18 hours at -80°C, sonicated with the SONIFIER250 (BRANSON), and milled the cell bodies. NaCl was added up to 0.5 M, and the supernatant was collected after centrifugation (10,000 g X 30 minutes). 200 ml of 0.25% Tween20/column buffer was added to the supernatant, and the solution was loaded on a column filled with 30 ml of amylose resin pre-equilibrated with 0.25% Tween20/column buffer (0.25% Tween20, 10 mM phosphate, 0.5 M NaCl, pH 7.2). With a flow rate of 1 ml/minute, after washing the column with 100 ml of 0.25% Tween20/column buffer and subsequently 150 ml of column buffer, the fusion protein bound to the amylose resin was eluted with 50 ml of the column buffer containing 10 mM maltose. The eluted fraction was concentrated to about 1 mg/ml with a ultrafilter (Amicon stirred-cell concentrator) as the concentrated fusion protein MBP-Meg-3.

The maltose binding protein portion of the fusion protein can be cleaved with an enzyme according to the following method. The protein solution is placed in a dialysis tube (fraction molecular weight: 3,500) and is dialyzed against the factor Xa buffer (20 mM Tris-HCl, 100 mM NaCl, 2 mM CaCl₂, 1 mM sodium azide). 10 µl factor Xa (200 µg/ml) is added to 200 µl (1 mg/ml) of the dialyzed solution, and the binding site between the maltose binding protein and the object protein is specifically cleaved by reacting at room temperature for 24 hours. After cleavage, the object protein can be obtained by purification methods utilizing gel filtration chromatography, ion exchange column chromatography, and such.

### Example 10: Production of polyclonal antibodies against MBP-Meg-3

The concentrated fusion protein MBP-Meg-3 obtained in Example 9 (10 mM sodium phosphate, 0.5 M NaCl, 10 mM maltose) is mixed with equal volumes of Freund's complete adjuvant, and was thoroughly emulsified. 0.5 ml of the emulsion was administered subcutaneously to the New Zealand white rabbit (female, about 4,000 g) (20 µg/animal). After priming, additional immunization were carried out with MBP-Meg-3 mixed with Freund's incomplete adjuvant, after 3 weeks (50 µg/animal), 5 weeks (50 µg/animal), 7 weeks (50 µg/animal), 9 weeks (100 µg/animal), and 11 weeks (200 µg/animal). 1 week after the third immunization, blood was collected to evaluate the antibody titer. The antibody titer was confirmed to have risen 204,800 folds. The antibody titer was measured by EIA using a 96 well plate coated with 50 ng/well antigen. 100 µl of successively diluted antisera was added to each well to perform the primary reaction, supernatant was discarded, and after washing, anti-rabbit IgG Fab'-HRP (IBL, Japan) was reacted. After washing, colored with OPD (Sigma, USA) to conduct measuring. In addition, the obtained antisera were confirmed to react specifically with the MBP-Meg-3 by Western blotting.

### Example 11: Reactivity examination of rabbit polyclonal anti-MBP-Meg-3 IgG

Using MBP-Meg-3, 3'-terminus c-myc tagged Meg-3, and MBP single-expression *E.coli* fracture solution as the antigen, reactivity of the rabbit IgG whose immunogen is MBP-Meg-3 was confirmed.

Cell fracture solution of the *E.coli* JM109, which was transformed with pMALc2/meg3 was used as MBP-Meg-3. Expression of 3'-terminal c-myc tagged Meg-3 was conducted using the primer (5'-GCGAATTCGAACTCAGTCTGCACCCCTGC-3'/ SEQ ID NO: 8), from which the stop codon was removed and to which a EcoRI recognition site was added, as the antisense primer instead of PCR A1 primer, and the synthesized fragment following the steps as in Example 9. The fragment was inserted into the mammalian expression vector pcDNA3.1 (Invitrogen) digested with EcoRI, and was expressed by *in vitro* transcription and translation (Promega) as c-myc tagged Meg-3 by utilizing the plasmid and the rabbit reticulocyte.

Samples were obtained by treating each of the protein solutions with the same amount of sample buffer (0.25% Tris-HCl, 2% SDS, 30% glycerine, 10% β-mercaptoethenol, 0.025% bromophenol blue) (Daiichi Chemicals) and heating them at 95°C for 5 minutes. The obtained samples were separated by SDS-polyacrylamide gel electrophoresis (SDS-PAGE) (Laemmli, U. K., Nature, 1970, 227: 680-685) using a gradient gel with gel concentration of 4-20% (Daiichi Chemicals).

Proteins separated by SDS-PAGE were blotted onto a polyvinylidene difluoride (PVDF) membrane (BioRad) by treatment in a blotting solution (25 mM Tris-HCl, 192 mM glycine, 20% methanol, pH 8.3) for 1 hour at constant voltage of 100 V. After washing the blotted PVDF membrane with distilled water, it was blocked in a 5% Block Ace solution in TTBS for 3 hours. Next, after washing the PVDF membrane with TTBS (20 mM Tris, 500 mM NaCl, 0.05% Tween 20, pH 7.5) it was reacted overnight at 4°C with a solution of rabbit polyclonal anti-MBP-Meg-3 IgG which is a primary antibody diluted with TTBS. Next, detection was carried out using Amplified Alkaline Phosphatase Immune Blot Kit (Biorad). In other words, after 1-hour incubation at room temperature with biotin-labeled goat anti-rabbit IgG diluted with TTBS, it was reacted with a previously prepared complex of streptavidin-biotin-labeled alkaline phosphatase for 1 hour by incubating streptavedin-biotin-labeled alkaline phosphatase at room temperature. By washing the PVDF membrane with TTBS and incubating it with a substrate (nitro blue tetrazolium and 5-bromo-4-chloro-3-indolyl phosphate, *p*-toluidine salt solution) at room temperature for approximately 30 minutes, the antibody bound to the primary antibody was visualized. The reaction was terminated by thorough reaction with distilled water.

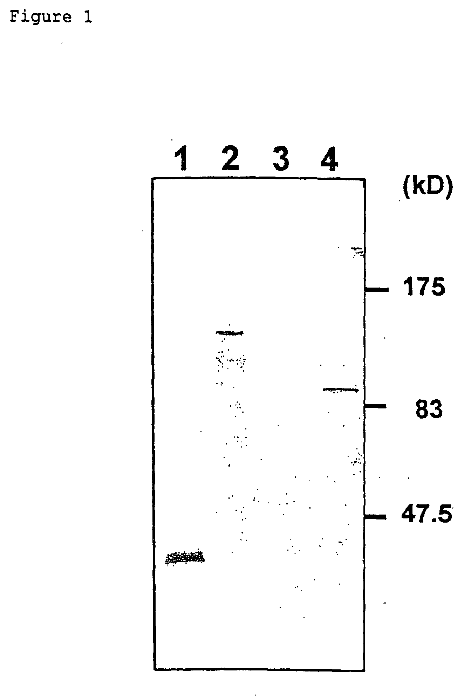

The results are shown in Figure 1. The band corresponding to MBP-Meg-3 was confirmed using the polyclonal anti-MBP-Meg-3 antibody of this invention obtained through Example 10. Therefore, it was shown that this polyclonal antibody was an antibody that specifically recognizes Meg-3.

### Example 12: Intracellular localization of Meg-3

Above pcDNA3.1 inserted with the ORF fragment of EcoRI fragment Meg-3 was transformed into the CHO cells and the 3'-terminus c-myc tagged Meg-3 was overexpressed. After 2 days, cells were fixed with 4% paraformaldehyde and 0.5% Triton X-100, reacted with anti-mouse c-myc antibody, and were reacted with FITC tagged anti-mouse antibody. Additionally, the nucleus was stained with Hoechst 33341, and was observed using the confocal laser microscope.

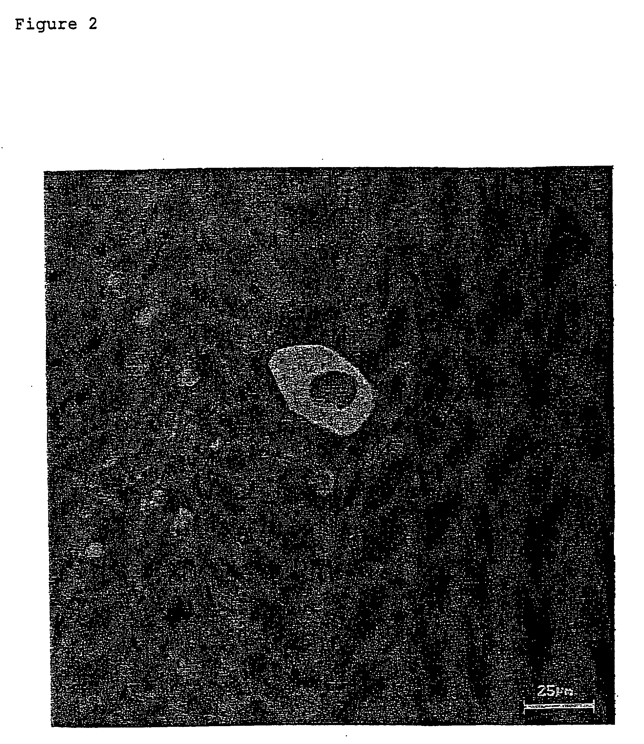

The result is shown in Figure 2. As predicted in the amino acid sequence analysis, it was confirmed that Meg-3 is localized in the cytoplasm.

### Industrial Applicability

The present invention provides a DNA expressed at high frequency in mesangial cells, a protein encoded by the DNA, etc. These are supposed to be closely related to mesangial cell-specific bioactivity, and useful for, for example, identifying mesangial cells, and detecting abnormalities in mesangial cells. Moreover, this protein would be helpful for clarifying the functions of mesangial cells and in turn, for investigating causes of diseases relating to mesangial cells. This invention is expectedly applicable to the treatment and diagnosis, and such of diseases relating to mesangial cells.

Specifically, the onset or progress of glomerulonephritis may be regulated, for example, by artificially adjusting Meg-3. Alternatively, quantification of mRNA and protein of Meg-3 in the body fluid and mesangial cells may enable diagnosis of renal diseases such as glomerulonephritis. In glomerulonephritis, functional abnormality is seen in the mesangium region, causing proliferation of mesangial cells or acceleration of matrix production from the cells. The possibility that Meg-3 is involved in these diseases is great.

Meg-3 of this invention and MEGSIN, which was previously reported by the present inventor, share common characteristics with regard to high levels of expression in the mesangial cells. However, the possibility that Meg-3 of this invention is an intracellular signal transduction substance including proline rich domain is suggested, whereas MEGSIN is a protein that is homologous to the SERPIN superfamily, which is a protease inhibitor. In addition, the observation that Meg-3 of this invention is expressed in a variety of tissues is different from the characteristic of MEGSIN, which is expressed specifically in mesangial cells. Therefore, Meg-3 of this invention may possibly be an important protein that supports the function of mesangial cells. This invention, which has elucidated the existence of such an important protein, has great significance.

## Claims

1. A protein comprising the amino acid sequence of SEQ ID NO: 2, or a protein comprising the amino acid sequence of SEQ ID NO: 2 in which one or more amino acids are replaced, deleted, added, and/or inserted, and being functionally equivalent to the protein comprising the amino acid sequence of SEQ ID NO: 2.

2. The protein of claim 1, wherein the protein comprises the amino acid sequence of SEQ ID NO: 2.

3. A DNA encoding the protein of claim 1.

4. The DNA of claim 3, wherein the DNA comprises the nucleotide sequence of SEQ ID NO: 1.

5. A DNA encoding the protein of claim 1 or functionally equivalent with these protein, the DNA hybridizing under stringent conditions with DNA comprising the nucleotide sequence of SEQ ID NO: 1.

6. A DNA hybridizing specifically with the DNA of claim 4 and having a chain length of at least 15 nucleotides.

7. An antisense DNA against the DNA of claim 4 or a portion thereof.

8. A vector comprising the DNA of any one of claim 3, claim 4 and claim 5.

9. A transformant expressibly carrying the DNA of any one of claim 3, claim 4 and claim 5.

10. A method for producing the protein of claim 1, the method comprising culturing the transformant of claim 9 and collecting an expression product of the DNA of any one of claim 3, claim 4 and claim 5.

11. A reagent for the detection of mesangial cells comprising the DNA of claim 6.

12. An antibody binding to the protein of claim 1.

13. The antibody of claim 12, wherein the antibody recognizes a portion of a protein comprising an amino acid sequence selected from the amino acid sequence of SEQ ID NO: 2.

14. The antibody of claim 13, wherein the antibody is a monoclonal antibody.

15. An immunoassay method for measuring the protein of claim 2 or a fragment thereof based on immunological binding of the antibody of any one of claim 13 or claim 14 to the protein of claim 2 or a fragment thereof.

16. A reagent for detecting the mesangial cell, the reagent comprising the antibody of any one of claim 12 to claim 14.

17. A method for detecting mesangial proliferative nephropathy, the method comprising measuring the protein of claim 2 or a fragment thereof contained in a biological sample and comparing the measured value with that obtained from a normal sample.

18. A transgenic nonhuman vertebrate in which the expression level of a gene encoding Meg-3 is modified.

19. The transgenic nonhuman vertebrate of claim 18, wherein the nonhuman vertebrate is a mouse.

20. The transgenic nonhuman vertebrate of claim 19, wherein the nonhuman vertebrate is a knockout mouse in which the expression of a gene encoding Meg-3 is inhibited.
